# EUROPEAN PATENT APPLICATION

(11) **EP 2 574 789 A2**
(43) Date of publication of application: **03.04.2013**
(21) Application number: 12186697.4
(22) Date of filing: 28.09.2012
(51) Int. Cl.: F04B 43/12, F16K 99/00, B01L 3/00

(54) **Fluidic Manifold**

(30) Priority: 30.09.2011 US 201113249470
(71) Applicant: DePuy Mitek, Inc., Raynham, MA 02767 (US)
(72) Inventor: Barry, Donald, Raynham, MA 02767 (US)
(74) Representative: Carpmaels & Ransford

(57) **Abstract**

A fluid management cassette system comprises a first and second opposed platen with first and second opposed films compressed between the platens. One or more channels formed one or both of the platens form pathways with the films through which fluids can be directed. Valves and other flow control mechanisms can be incorporated.

## Description

### Background

The present application relates to fluid manifold and more particularly to a manifold and methods associated therewith in which fluid flow paths are created between a pair of films.

In the fields of biomedical processing and analysis, fluids are typically manipulated and conducted between various locations via tubing that requires connections and interfaces to control devices. These fluidic systems comprise one or more removable tubing harnesses and the processing machine. The tubing harness or cassette is usually a single use disposable in order to guarantee purity or sterility. The tubing harnesses are loaded onto reusable processing machine by an operator who must connect the control devices such as pumps and valve to the harness. Depending on the complexity of the harness, significant training, time, and potential for errors exist in the use of tubing harnesses. There are multiple connections between tubing sections and components that have the potential for leaking. Moreover, the material and assembly cost of a disposable tubing harness or cassette is significant and affects the commercial adoption of products and procedures.

There have been many systems invented to assist in this loading process to save time, and avoid errors, such as custom cassettes and different types of tubing holders. Manifold systems have been invented where the tubing function is incorporated into channels formed in a rigid member. Typically, manifold, cassette and tubing holder approaches add material costs above the basic tubing harnesses. Additionally cassettes and tubing holders provide a single fluidic configuration that is defined at manufacturing time. Manifolds provide somewhat increased configuration flexibility but are limited to the pathways incorporated into the design.

### Summary of the Invention

A fluid management cassette system according to the present invention comprises a first platen and a second platen opposed to the first platen. A first film and a second film, in opposed relation to each other, are compressed between the first platen and the second platen. One or more channels are formed in at least one of the first platen and second platen whereby fluid can be directed through a pathway formed between the first film and second film at the one or more channels.

Preferably, the first film has an inward face facing the second film and the second film has an inward face facing the first film and wherein the first film inward face and the second film inward face are sterile. Preferably, they are disposable and replaceable. Also preferably, they are sealed about an extent of their peripheries so as to form a bag.

Preferably, there is a valve comprising a valve element movable into the channel against one of the first or second films. In one aspect of the invention, the pathway is blocked when the valve element is moved fully into the channel. The valve element can be movable partially into the channel to effect a flow restriction in the pathway without total blockage.

In one aspect of the invention, at least one of the first platen and second platen comprises a matrix of segments movable from an extended position toward the other of the first platen and second platen to a retracted position away from the other of the first platen and second platen with the channel formed by an arrangement of the segments in their retracted positions.

A flow control valve can be effected in the channel via one or more of the segments moveable into the channel.

In one aspect of the invention a fluid pump formed in at least one of the first and second platens. For instance a chamber along one of the one or more channels and having a check valve forming an inlet into the chamber and arranged to allow flow into the chamber can be employed with a pump element at the chamber arranged to apply pressure against one of the first and second films at the chamber. The pressure drives flow out of the chamber and the check valve prevents the flow from going backward. Preferably another check valve is provided at the outlet to prevent backflow into the chamber from the outlet. Alternatively, the pump can comprise a positive displacement element movable along one of the one or more channels to effect a peristaltic pumping action.

A method according to the present invention provides for managing a fluid flow. The method comprises trapping opposed first and second films between opposed first and second platens; forming a fluid pathway between the first and second films via a channel formed into at least one of the first and second platens; and flowing fluid through the pathway.

Preferably, the first and second films have surfaces at the pathway and said surfaces are sterile prior to the step of flowing fluid through the pathway.

Preferably, the step of impeding flow of fluid through the pathway comprises moving a valve element against at least one of the first and second films and into the channel to obstruct flow through the pathway. The movement of the valve element into the channel can completely block flow through the pathway or merely throttle the flow by partially blocking the pathway.

In one aspect of the invention, at least one of the first and second platens comprises a matrix of segments movable from an extended position toward the other of the first platen and second platen to a retracted position away from the other of the first platen and second platen and the channel is formed by an arrangement of the segments in their retracted positions.

Pumping fluid along the channel can be effected by engaging segments along the channel in a wave pattern to induce flow along the channel.

### Brief Description of the Drawings

FIG. 1 is an exploded perspective view of a fluid manifold according to the present invention;

FIG. 2 is a perspective view of the fluid manifold of FIG. 1;

FIG. 3 is a sectional view along lines 3 -- 3 of FIG. 1, with its platens shown in a spaced apart configuration with the bag in between;

FIG. 4 is a sectional as in FIG. 3 but with the platens pressed together and with fluid pathways carrying fluid;

FIG. 5 is a sectional view along lines 5 -- 5 of FIG. 1 showing the pumping chamber;

FIG. 6 is a sectional view taken along lines 6 -- 6 of FIG. 1 showing the check valve ;

FIG. 7 is a perspective view of a bottom block of an alternative fluid manifold according to the present invention and which has reconfigurable fluid channels; and

FIGS. 8A to D are side elevation views in time sequence of a flow path of the manifold of FIG. 7 showing peristaltic pumping action.

### Detailed Description

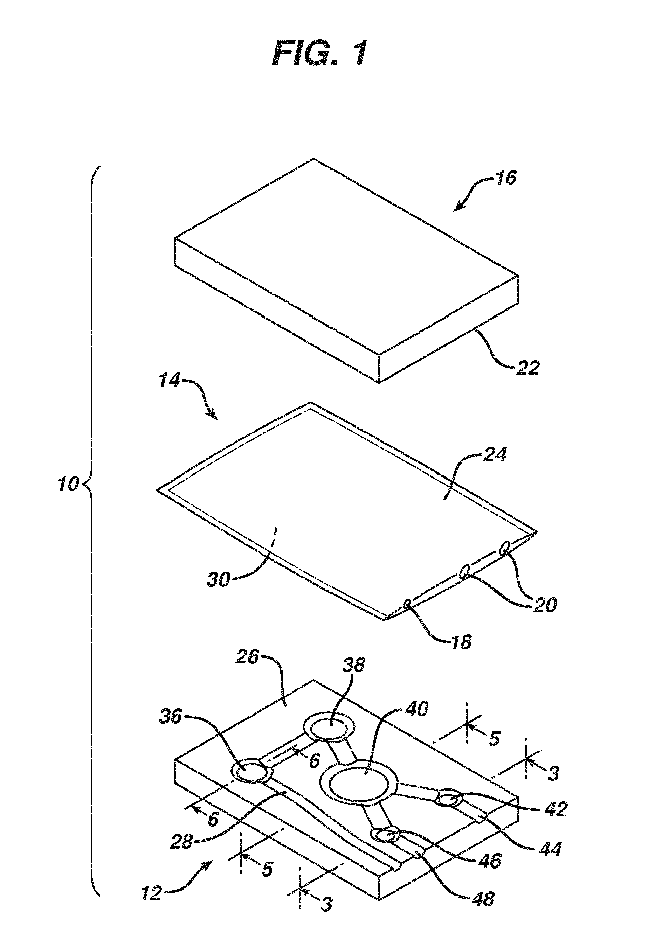

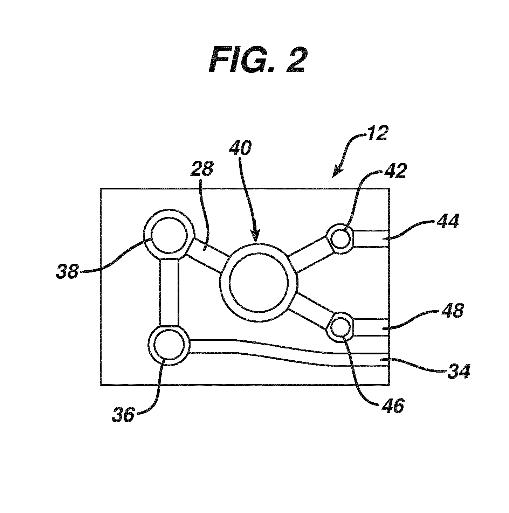

FIGS. 1 and 2 depict a fluidic manifold system 10 according to the present invention comprising in gross a shaped bottom block 12, a compliant fluid bag 14 and an unshaped top block 16. The bag 14 is a fluid or gas containing compartment preferably formed by folding or sealing films of a biocompatible material such as, and without limitation, PVC, PolyUrethanes, Polyethelynes, Silicones and Polypropylenes and installing one or more inlet connections 18 and one or more outlet connections 20. Other arrangements are possible; for instance fluid carrying tubes (not shown in FIGS. 1 and 2) can be provided already connected to the bag 14 and carrying their own connectors for connection to fluid sources or dispensing apparatus. Either or both of the connectors and tubing can be formed integral with the bag 14. The top block 16 has a flat lower surface 22 which contacts a top film 24 forming the bag 14. The bottom block 12 has an upper surface 26 having channels 28 formed thereon and which contacts a lower film 30 forming the bag 14. Rather than being flat, the top block lower surface 22 could have mating channels (not shown) in registry with the channels 28 of the bottom block 12 or which are independently positioned with respect to those channels 28.

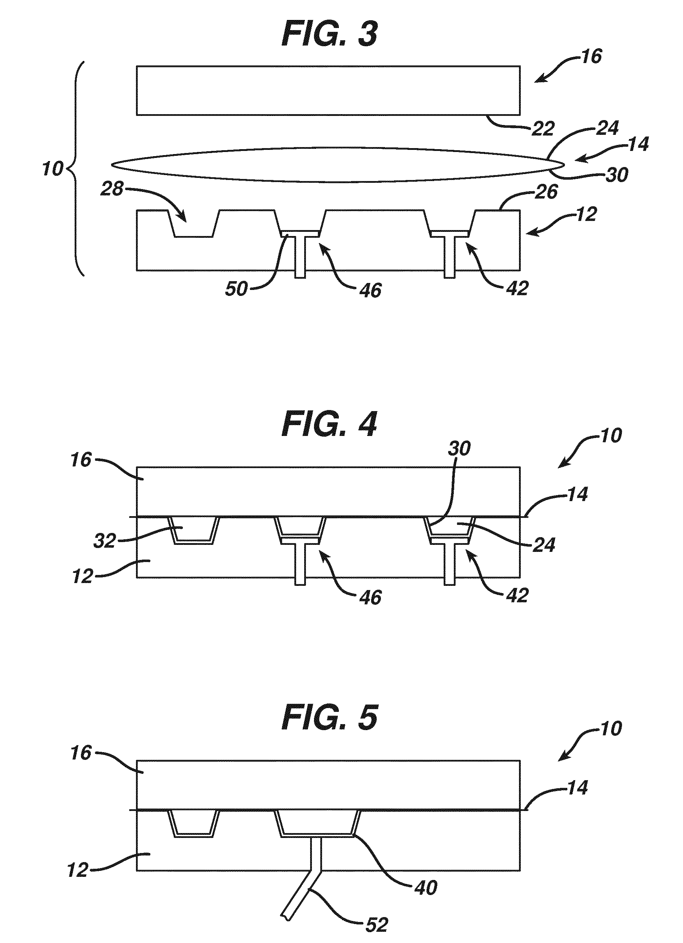

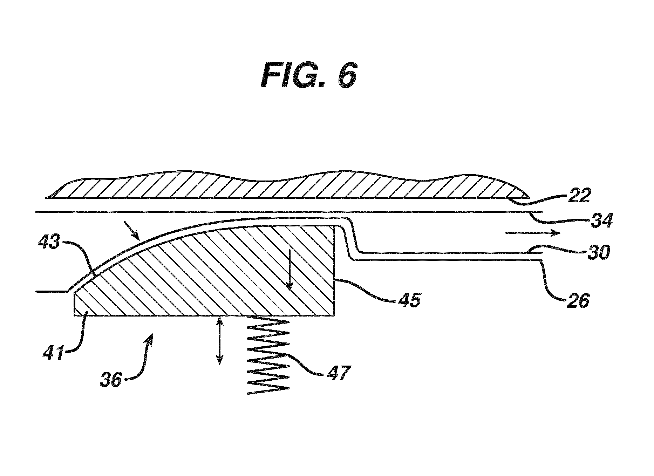

Turning also now to FIGS. 3 to 6 the interaction between the bag 14, top and bottom blocks 16 and 12 and the channels 28 form fluid pathways 32 through the bag 14. The bag is trapped between the blocks 12 and 16 which act as platens compressing the bag in some areas and not where the channels 28 are present. The bag is flexible to act as a gasket sealing the pathways 32. Preferably the material of the bag 14 itself has some resiliency to enhance its action in sealing as a gasket. While the blocks 12 and 16 are preferably rigid for durability one or both could be formed of or have attached thereto a resilient material to assist in sealing. This could be conveniently be applied to the flat surface 22 and be replaceable when worn or when different resiliency characteristics are desired.

The arrangement (best seen in FIGS. 1 and 2) of the channels 28 determines the fluid pathways 32. The fluid manifold system 10 shown has an inlet 34 and the channel 28 leads therefrom to a check valve 36. From the check valve the channel 28 leads to a variable flow valve 38 and then to a pumping chamber 40. From the pumping chamber 40 two channels 28 lead to a first outlet valve 42 and outlet 44 and to a second outlet valve 46 and second outlet 48. The inlet 34 corresponds with the bag inlet connection 18 and the outlets 44 and 48 with the bag outlet connections 20, preferably in such an arrangement that the bag 14 only fits in one orientation to prevent incorrect placement thereof.

The outlet valves 42 and 46 are simple plungers 50 which block the pathway 32 to prevent flow therethrough. The variable flow valve 38 also comprises a plunger which can be adjusted to partially block the pathway 32. The check valve 36 comprises a valve body 41 having a curved inlet surface 43 and a flat outlet surface 45 and which is biased closed by a spring 47. Flow against the inlet surface 43 provides pressure to overcome the bias of the spring 47 an move the valve body 41 open to allow flow. Flow against the outlet surface 45 will not generate enough pressure to overcome the bias.

By alternately applying suction and pressure to the pumping chamber 40 through an air line 52 a pumping effect can be achieved and the check valve 36 will cause the flow to be toward the outlets 44 and 48. An additional check valve (not shown) may be desirable on the outlet from the pumping chamber 40 to prevent back flow into it and to enhance the pumping efficiency. A mechanical solution may also be employed such as a piston (not shown) which moves against the film 30 at the pumping chamber 40. Alternatively, rollers (not shown) or other positive displacement type drivers could be applied to a section of the pathway 32 to effect a peristaltic pumping action.

Pressure sensors (not shown) can be effected by placing a force measuring sensor, such as a strain gauge, into one of the blocks 12 or 16 at the pathways 32. The force applied thereto by the fluid within the pathway 32 can be calibrated to indicate pressure. Flow volume can be determined with multiple pressure sensors and calculating pressure drop through a section of the pathway 32 between them having known dimensions, perhaps assisted with a metering constriction of the pathway 32. Flow measurements can also be calibrated with an external flowmeter.

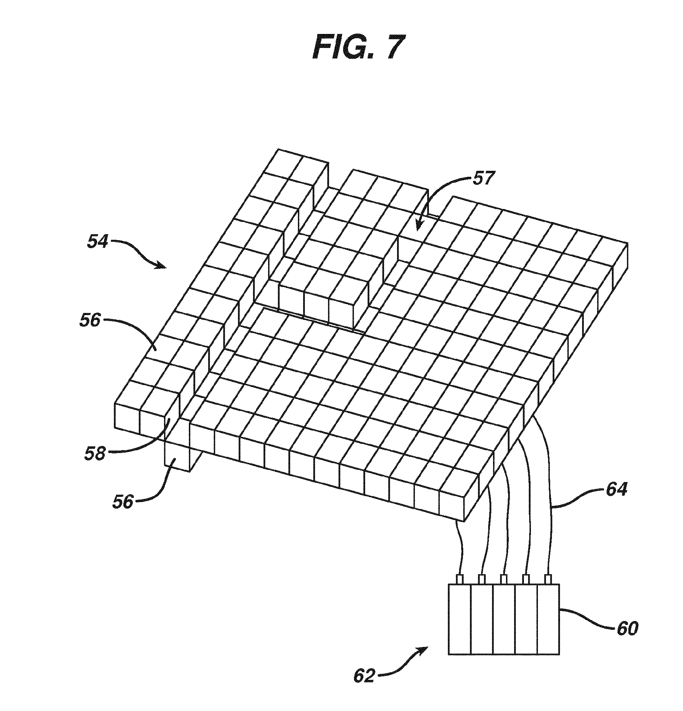

Flexibility in fluid management can be achieved in a second embodiment of a block 54 (see FIG. 7) which replaces the block 12. The block 54 comprises a matrix of sections 56 each of which is capable of independent actuation, preferably variable, such that channels 58 can be created by retracting certain sections 56 and valves 57 by extending sections 56 within the channels 58. The valves 57 can be flow control valves by only partially extending a section 56. Only a simplified version is shown for illustrating the principle with a channel 58 formed by retracted sections 56 and extending from an inlet 51 and branching to two outlets 53 and 55, with the outlet 55 blocked by the valve 57 which comprises one of the sections 56 being extended into the channel 58. The entire operational portion of the block 54 can comprise the sections 56 or in can have some channels and features permanently formed therein with only some portions of the block 54 comprised of the sections 56. The sections 56 can be used to effect valves and pumps and other flow control items.

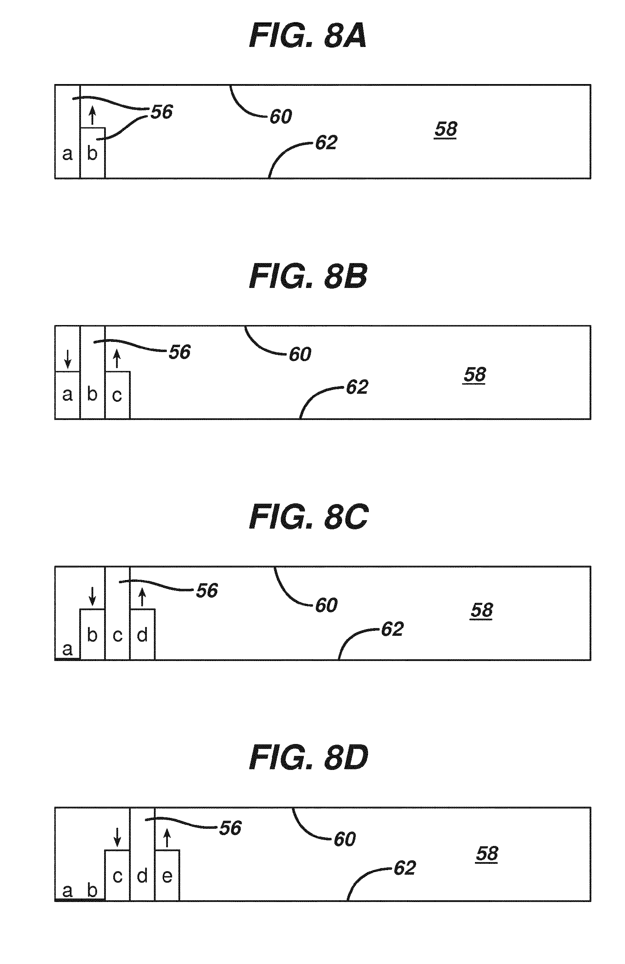

For instance, peristaltic pumping action could be effected by actuating sections 56 within a channel 58 in waves as illustrated in FIGS. 8A to D, where each of the sections 56 are coded with a letter for illustration, and they operate between a first rigid surface 60 and a second surface 62 formed of the individual sections 56 (the bag 14 is omitted for clarity but would be positioned between the surfaces 60 and 62). At time T₁ (FIG. 8A) section 56 coded "a" is fully actuated and that coded "b" is on its way up. At a slightly later time T₂ (FIG. 8B) the section 56 coded "a" is on its way down, "b" is fully actuated and "c" is on its way up. By time T3 (FIG. 8C) "a" is fully retracted, "b" is on the way down, "c" is fully actuated" and "d" is on the way up. By time T4 (FIG. 8D) both "a" and "c" are fully retracted etc. In this fashion a wave of flow is created through the channel 58.

Preferably each section is independently controlled and actuated via a control system and independent linear actuators or solenoids 60. Other suitable mechanisms for independently controlling movement of the sections 56 are contemplated, such as pneumatic or hydraulic cylinders, mechanical lever actuators, electric motors, etc. In some applications the desired sections may be smaller than reasonable cost actuators, in which instance the actuators 60 can be arranged into a matrix 62 (only a small portion of such matrix being illustrated in FIG. 7), preferably matching the matrix of sections 56, and a series of movement actuation lines 64, such as Bowden cables, can transmit the movement of the actuator to the individual sections 56. Thus, the actuators would not need to be miniaturized to meet the dimensions of the sections 56.

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims or the equivalents thereof.

## Claims

1. A fluid management cassette system comprising;
a first platen and a second platen opposed to the first platen;
a first film and a second film in opposed relation to each other and compressed between the first platen and the second platen; and
one or more channels formed in at least one of the first platen and second platen whereby fluid can be directed through a pathway formed between the first film and second film at the one or more channels.

2. The fluid management cassette system of claim 1 wherein the first film has an inward face facing the second film and the second film has an inward face facing the first film and wherein the first film inward face and the second film inward face are sterile.

3. The fluid management cassette system of claim 1 and further comprising a valve comprising a valve element movable into the channel against one of the first or second films.

4. A method of managing a fluid flow comprising:
trapping opposed first and second films between opposed first and second platens;
forming a fluid pathway between the first and second films via a channel formed into at least one of the first and second platens; and
flowing fluid through the pathway.

5. The method of claim 4 wherein the first and second films have surfaces at the pathway and said surfaces are sterile prior to the step of flowing fluid through the pathway.

6. The method of claim 4 and further comprising the step of impeding flow of fluid through the pathway by moving a valve element against at least one of the first and second films and into the channel to obstruct flow through the pathway.

7. The fluid management cassette system of claim 3 or the method of claim 6 wherein:
the pathway is blocked when the valve element is moved fully into the channel; or
a flow restriction is effected in the pathway when the valve element is moved partially into the channel.

8. The fluid management cassette system of claim 1 or the method of claim 4 wherein at least one of the first and second platens comprises a matrix of segments movable from an extended position toward the other of the first platen and second platen to a retracted position away from the other of the first platen and second platen and the channel is formed by an arrangement of the segments in their retracted positions.

9. The fluid management cassette system or the method of claim 8 wherein a flow control valve in the channel comprises one or more of the segments moveable into the channel.

10. The method of claim 9 further comprising pumping fluid along the channel by engaging segments along the channel in a wave pattern to induce flow along the channel.

11. The fluid management cassette system of claim 1 wherein the first film and second film are disposable and replaceable.

12. The fluid management cassette system of claim 1 wherein the first film and second film are sealed about an extent of their peripheries so as to form a bag.

13. The fluid management cassette system of claim 1 and further comprising a fluid pump formed in at least one of the first and second platens.

14. The fluid management cassette system of claim 13 wherein the pump comprises a chamber along one of the one or more channels, a check valve forming an inlet from the one or more channel into the chamber and arranged to allow flow into the chamber, a pump element at the chamber arranged to apply pressure against one of the first and second films at the chamber whereby the pressure drives flow out of the chamber.

15. The fluid management cassette system of claim 13 wherein the pump comprises a positive displacement element movable along one of the one or more channels to effect a peristaltic pumping action.
